Europäisches Patentamt

**European Patent Office**

Office européen des brevets

(19)

(11) Veröffentlichungsnummer: **0 200 987**
**B1**

(12) **EUROPÄISCHE PATENTSCHRIFT**

(45) Veröffentlichungstag der Patentschrift:
**12.07.89**

(21) Anmeldenummer: **86105540.8**

(22) Anmeldetag: **22.04.86**

(51) Int. Cl.⁴: **A61B 6/00**, A61B 6/04

(54) **Röntgengerät mit Federgewichtsausgleich.**

(30) Priorität: **06.05.85 DE 8513305 U**

(43) Veröffentlichungstag der Anmeldung:
**17.12.86 Patentblatt 86/46**

(45) Bekanntmachung des Hinweises auf die Patenterteilung:
**12.07.89 Patentblatt 89/28**

(84) Benannte Vertragsstaaten:
**DE FR GB**

(56) Entgegenhaltungen:
**DE-B- 1 047 988**
**DE-C- 968 324**
**DE-U- 1 728 886**

(73) Patentinhaber: **Siemens Aktiengesellschaft Berlin und München, Wittelsbacherplatz 2, D-8000 München 2(DE)**

(72) Erfinder: **Hahn, Alfred, Bunsenstrasse 64, D-8520 Erlangen(DE)**
Erfinder: **Kamm, Bodo, Speckweg 46, D-8521 Möhrendorf(DE)**
Erfinder: **Weiss, Karl, Im Föhrenwald 23, D-8520 Buckenhof(DE)**
Erfinder: **Dummert, Johannes, Volckamerstrasse 34, D-8520 Erlangen(DE)**

## Beschreibung

Die Erfindung betrifft ein Röntgengerät mit einem Gerätefuß, einem kippbaren Teil, einem auf dem kippbaren Teil entlang Führungsschienen laufenden, verschiebbaren Wagen für Röntgenteile mit Federgewichtsausgleich, mit je nach Gerätelage motorisch verstellbaren Ausgleichskräften.

Aus der DE-PS 968 324 ist ein derartiges Röntgengerät mit Federgewichtsausgleich bekannt, bei dem das an einem Lasthebel wirkende Gewicht durch die Gegenkraft einer einzigen am Lasthebel angreifenden Feder ausgeglichen wird. Zur Einstellung der je nach Schräglage der Einrichtung erforderlichen unterschiedlichen Gegenkraft wird der Angriffspunkt der Feder über ein Kurbelgetriebe verstellt. Da der Verstellbereich der Kurbel verhältnismäßig klein, die zu bewegenden Teile und ihre Gewichte aber recht groß sind, läßt sich der Ausgleich nicht mit der nötigen Zuverlässigkeit bewerkstelligen. Weiterhin wird zur Erzielung einer etwa gleichbleibenden Federkraft bei unterschiedlicher Positionierung des gewichtsausgeglichenen Wagens mit einem Zielgerät eine Kurvenscheibe benötigt. Dadurch ist aber eine genau konstante Federkraft nicht erzielbar. Ferner sind derartige Kurvenscheiben sehr schwierig herzustellen.

Deshalb ist in der DE-AS 1 047 988 vorgeschlagen, den Angriffspunkt des Federgewichtsausgleiches mittels einer durch einen Verstellmotor angetriebenen Spindel zu verstellen, so daß entsprechend der Schräglage des Gerätes ein Ausgleich erfolgen kann. Eine Anpassung der Gegenkraft zum auszugleichenden Gewicht für erforderliche Längsbewegungen in Richtung des Federausgleiches wird auch hier durch Kurvenglieder erreicht.

Die Erfindung geht von der Aufgabe aus, ein Röntgengerät der eingangs genannten Art zu schaffen, das sich durch eine geringe Baugröße bei möglichst großem Hub auszeichnet und bei dem für die Einstellung des Federgewichtsausgleiches in Abhängigkeit vom Hub, der Schräglage und des auszugleichenden Gewichtes nur ein Stellglied für den Federgewichtsausgleich vorgesehen ist.

Die Aufgabe wird erfindungsgemäß durch die in dem kennzeichnenden Teil des Patentanspruches 1 angegebenen Merkmale gelöst. Dadurch läßt sich erreichen, daß in Abhängigkeit von der Schräglage der Angriffspunkt der motorisch verstellbaren Mutter und somit die durch die Feder erzeugte Gegenkraft verstellbar sind, wobei aber unabhängig vom Hub das durch die Feder hervorgerufene Drehmoment am Lasthebel konstant bleibt.

Ein vorteilhafter Aufbau ergibt sich, wenn der Federgewichtsausgleich für einen Zielgerätewagen im Innern eines Führungsturmes angeordnet ist. Ein Ausgleich in Längsrichtung eines Tisches mit großem Hub läßt sich erreichen, wenn der Federgewichtsausgleich in einem motorisch verfahrbaren Längswagen innerhalb eines Kipptisches angeordnet ist. Eine einfache Steuerung erhält man, wenn an der Gewindespindel ein Motor angebracht ist, der mit einer Regelschaltung verbunden ist, und wenn dem Gerätefuß ein Winkelaufnehmer und der Gewindespindel ein Längenaufnehmer zugeordnet ist, die mit der Regelschaltung verbunden sind, so daß die durch die motorgetriebene Gewindespindel einstellbare, auszugleichende Kraft mit einer sinusförmigen Winkelfunktion verläuft. Der Hub des auf dem Längslaufwagen angeordneten Federgewichtsausgleiches läßt sich erhöhen, wenn an dem Lasthebel ein weiterer Aufnehmer angebracht ist, der ein den Abweichungen des Lasthebels aus der Mittelstellung entsprechendes Signal einer weiteren Regelschaltung zuführt, die den Motor für den Längswagen derart steuert, daß die Abweichung ausgeglichen wird.

Nachfolgend ist die Erfindung anhand eines in der Zeichnung dargestellten Ausführungsbeispieles näher erläutert. Es zeigen:

Fig. 1 eine Prinzipskizze eines erfindungsgemäßen Röntgengerätes,

Fig. 2 einen Schnit durch den Längstisch des Röntgendiagnostikgerätes gemäß Figur 1, und

Fig. 3 ein Blockschaltbild der Steuerung des Federgewichtsausgleiches eines erfindungsgemäßen Röntgengerätes.

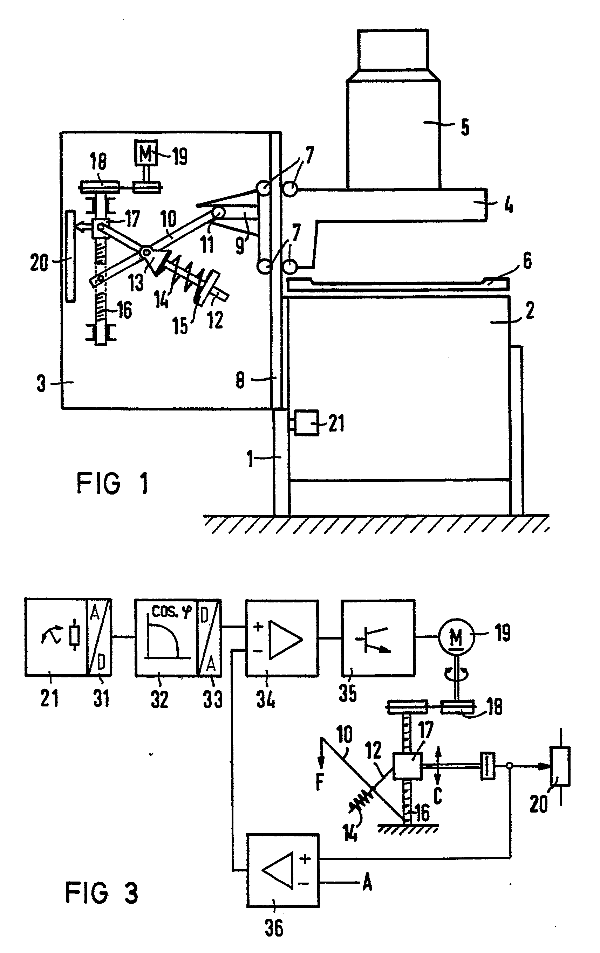

In der Figur 1 ist ein Röntgengerät mit einem an einem Gerätefuß 1 drehbar befestigten Kipptisch 2 dargestellt, an dem ein Führungsturm 3 für einen Zielgerätewagen 4 angebracht ist, der das Zielgerät 5 mit Röntgenbildverstärker-Fernsehkette trägt. Auf dem Kipptisch 2 ist eine Patientenlagerungsplatte 6 beweglich angebracht. Der Führungsturm 3 ist zur besseren Übersicht um 90° geschwenkt dargestellt.

In dem Führungsturm 3 läuft der Zielgerätewagen 4, gestützt durch Rollen 7, entlang Führungsschienen 8. An seinem inneren Teil weist der Zielgerätewagen 4 eine im wesentlichen senkrecht zu den Führungsschienen 8 verlaufende, schlitzförmige Öffnung 9 auf, in die eine an einem einarmigen Lasthebel 10 angebrachte Rolle 11 gleitet. Der Lasthebel 10 ist an seinem anderen Ende drehbar mit dem Führungsturm 3 verbunden. Zwischen den beiden Enden ist der Lasthebel 10 mit einer an einer Stange 12 gleitend gelagerten Muffe 13 schwenkbar verbunden, die sich über eine Feder 14 an einem an dem Zielgerätewagen zugewandten Ende der Stange 12 angebrachten Befestigungsglied 15 abstützt. Das andere Ende der Stange 12 ist mit einer durch eine Gewindespindel 16 verstellbaren Mutter 17 gelenkig verbunden. Die Gewindespindel 16 wird über ein Untersetzungsgetriebe 18 von einem Motor 19 angetrieben. Sie verläuft parallel zu den Führungsschienen 8 und geht durch den Drehpunkt des Lasthebels 10. Ein Längenaufnehmer 20, der mit der Mutter 17 gekoppelt ist, stellt die augenblickliche Lage der Mutter 17 fest. An dem Kipptisch 2 ist ein Winkelaufnehmer 21 angebracht, der mit dem Gerätefuß gekuppelt ist und die Schräglage des Kipptisches 2 erfaßt.

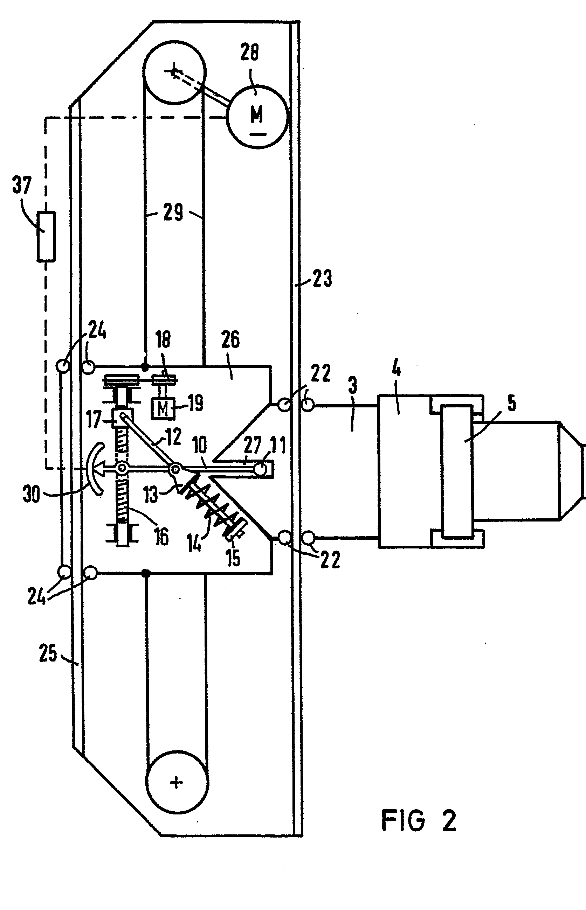

In der Figur 2 ist ein weiterer Anwendungsfall des erfindungsgemäßen Federgewichtsausgleiches dargestellt, der den auf Rollen 22 entlang Führungsschienen 23 laufenden Führungsturm 3 mit Zielgerät 5 ausgleicht. Der Federgewichtsausgleich 10 bis 20 ist auf einem auf Rollen 24 in Führungsschienen 25 laufenden Längswagen 26 angeordnet. Er greift mit seinem Lasthebel 10 in eine an dem Führungsturm 3 angebrachte schlitzförmige Öffnung 27. Über eine durch einen Motor 28 betriebene Kette 29, die mit dem Längswagen 26 verbunden ist, wird dieser derart verstellt, daß der Lasthebel 10 bei der gewünschten Position des Führungsturmes 3 immer, wie dargestellt, in seiner Mittelstellung ausgerichtet ist. Die Stellung des Lasthebels 10 wird durch einen mit diesem gekoppelten Aufnehmer 30 ermittelt.

Anhand der Figur 3, in der ein Blockschaltbild der Steuerung des Federgewichtsausgleiches dargestellt ist, wird dessen Funktionsweise näher erläutert. Der Winkelaufnehmer 21 liefert über einen A/D-Wandler 31 ein dem Winkel des Kipptisches 2 entsprechendes Signal einem Funktionsspeicher 32, in dem die Sinus- bzw. Cosinusfunktion enthalten ist. Für den Federgewichtsausgleich gemäß Figur 1 wird die Cosinusfunktion und für den Federgewichtsausgleich gemäß Figur 2 die Sinusfunktion benötigt, da bei einem Umlegewinkel von ± 90° ein Ausgleich nur im Fall 1 in einer vom Kipptisch 2 abgewandten Richtung und im Fall 2 in beiden Richtungen erfolgen muß.

Über einen D/A-Wandler 33 wird das Ausgangssignal des Funktionsspeicher 32 einer ersten Vergleichsstufe 34 zugeführt, die mit einer Verstärkerstufe 35 für den Motor 19 verbunden ist. Der Motor 19 ist über das Untersetzungsgetriebe 18, die Gewindespindel 16 und die Mutter 17 mechanisch mit dem Längenaufnehmer 20 verbunden, der ein der Stellung der Mutter 17 proportionales Signal einer zweiten Vergleichsstufe 36 werden einstellbare Werte A zugeführt, die den Ausbaustufen und damit dem Gewicht des Zielgerätes 5 entsprechen und somit den Sollwert für die einzustellende Gegenkraft darstellen. Die zweite Vergleichsstufe 36 ist mit dem zweiten Eingang der ersten Vergleichsstufe 34 verbunden und liefert ihr eine der Abweichung vom Sollwert der auszugleichenden Kraft entsprechende Regelgröße. Diese wird mit der Winkelfunktion verglichen und die Mutte 17 durch den Motor 19 entsprechend nachgestellt, so daß die am Ende des Lasthebels 10 wirkende Gegenkraft das Gewicht des auszugleichenden Wagens vollends kompensiert.

Da aufgrund einer kompakten Bauweise der Hub des Federgewichtsausgleiches begrenzt ist, wird bei dem Ausführungsbeispiel gemäß Figur 2 durch den verfahrbaren Längswagen 26 dieser erhöht. Verschiebt beispielsweise die Untersuchungsperson am Kommandoarm des Zielgerätes den Führungsturm, so wird der Lasthebel 10 aus seiner Mittelstellung heraus bewegt. Diese Positionsänderung wird durch den Aufnehmer 30 erkannt, der ein Signal einer weiteren Regelschaltung 37 zuführt, die daraufhin den Motor 28 einschaltet, so daß der Längswagen 26 so lange nachgeführt wird, bis der Lasthebel 10 sich wieder in seiner dargestellten Mittelstellung befindet. Dadurch stehen genügend Reserven bei der Nachführung zur Verfügung, auch wenn die Untersuchungsperson eine schnelle Bewegung durchführen sollte. Die Einstellung des Federgewichtsausgleiches erfolgt in der gleichen Weise wie er anhand der Figur 3 beschrieben wurde.

Aufgrund der geometrischen Anordnung des Lasthebels 10, der Stange 12 und der Gewindespindel 16 wird erreicht, daß nach Einstellung der Mutter 17 durch die Gewindespindel 16 und dadurch erfolgten Ausgleiches des Gewichtes des Wagens am Angriffspunkt der Last, an der Rolle 11 des Lasthebels 10, in jeder Stellung des Lasthebels 10 das gleiche Moment auftritt, so daß die Ausgleichskraft konstant bleibt.

**Patentansprüche**

1. Röntgengerät mit einem Gerätefuß (1), einem kippbaren Teil (2, 3, 26), einem auf dem kippbaren Teil (2, 3, 26) entlang Führungsschienen (8, 23) laufenden, verschiebbaren Wagen (3, 4) für Röntgenteile mit Federgewichtsausgleich, mit je nach Gerätelage motorisch verstellbaren Ausgleichskräften, dadurch gekennzeichnet, daß an dem Wagen (3, 4) wenigstens eine im wesentlichen senkrecht zu den Führungsschienen (8, 23) verlaufenden, schlitzförmige Öffnung (9, 27) angebracht ist, in der das eine Ende eines am kippbaren Teil (2, 3, 26) angelenkten einarmigen Lasthebels (10) verschiebbar ist, daß der Lasthebel (10) zwischen seinen Enden mit einer an einer Stange (12) gleitend gelagerten Muffe (13) schwenkbar verbunden ist, die sich mit einer Feder (14) an dem dem Wagen (3, 4) zugewandten Ende der Stange (12) abstützt, deren anderes Ende an einer durch eine motorisch betätigbare Gewindespindel (16) verstellbaren Mutter (17) angelenkt ist, wobei die Gewindespindel (16) im wesentlichen parallel zu den Führungsschienen (8, 23) angeordnet ist.

2. Röntgengerät nach Anspruch 1, dadurch gekennzeichnet, daß der Federgewichtsausgleich für einen Zielgerätewagen (4) im Inneren eines Führungsturmes (3) angeordnet ist.

3. Röntgengerät nach Anspruch 1, dadurch gekennzeichnet, daß der Federgewichtsausgleich in einem motorisch verfahrbaren Längswagen (26) innerhalb eines Kipptisches (2) angeordnet ist.

4. Röntgengerät nach einem der Ansprüche 1 bis 3, dadurch gekennzeichnet, daß an der Gewindespindel (16) ein Motor (19) angebracht ist, der mit einer Regelschaltung (31 bis 36) verbunden ist, und daß dem Gerätefuß (1) ein Winkelaufnehmer (21) und der Gewindespindel (16) ein Längenaufnehmer (20) zugeordnet ist, die mit der Regelschaltung (31 bis 36) verbunden sind, so daß die durch die motorgetriebene Gewindespindel (16) einstellbare, auszugleichende Kraft mit einer sinusförmigen Winkelfunktion verläuft.

5. Röntgengerät nach Anspruch 3 oder 4, dadurch gekennzeichnet , daß an dem Lasthebel (10) ein weiterer Aufnehmer (30) angebracht ist, der ein den Abweichungen des Lasthebels (10) aus der Mittelstellung entsprechendes Signal einer weiteren Regelschaltung (37) zuführt, die den Motor (28) für den Längswagen (26) derart steuert, daß die Abweichung ausgeglichen wird.

## Revendications

1. Appareil radiologique comportant un pied (1), une partie basculante (2, 3, 26), un chariot mobile (3, 4) servant à supporter des éléments radiologiques et circulant sur la partie basculante (2, 3, 26) le long de rails de guidage (8, 23) et comportant un compensateur du poids à ressort, et dans lequel des forces d'équilibrage sont réglables au moyen d'un moteur en fonction de la position de l'appareil, caractérisé par le fait que dans le chariot (3, 4) se trouve ménagée au moins une ouverture en forme de fente (9, 27), qui s'étend sensiblement perpendiculairement aux rails de guidage (8, 23) et dans laquelle peut se déplacer une extrémité d'un levier de charge (10) à un seul bras, articulé sur la partie basculante (2, 3, 26), que le levier de charge (10) est relié de manière à pouvoir pivoter, entre ses extrémités, à un manchon (13), qui est monté de manière à glisser sur une barre (12) et prend appui, au moyen d'un ressort (14), sur l'extrémité, tournée vers le chariot (3, 4), de la barre (12), dont l'autre extrémité est articulée sur un écrou (17) déplaçable à l'aide d'une broche filetée (16) pouvant être actionnée par un moteur, la broche filetée (16) étant sensiblement parallèle aux rails de guidage (8, 23).

2. Appareil radiologique suivant la revendication 1, caractérisé par le fait que le compensateur du poids à ressort prévu pour l'équilibrage d'un chariot (4) d'un sélecteur est disposé à l'intérieur d'une colonne de guidage (3).

3. Appareil radiologique suivant la revendication 1, caractérisé par le fait que le compensateur du poids à ressort est disposé dans un chariot longitudinal (26), déplaçable à l'aide d'un moteur, à l'intérieur d'une table basculante (2).

4. Appareil radiologique suivant l'une des revendications 1 à 3, caractérisé par le fait que sur la broche filetée (16) est monté un moteur (19), qui est relié à un circuit de régulation (31 à 36), et qu'un capteur angulaire (21) est associé au pied (1) de l'appareil et qu'un capteur de longueur (20) est associé à la broche filetée (16), ces capteurs étant reliés au circuit de régulation (31 à 36) de manière que la force devant être équilibrée, qui est réglable à l'aide de la broche filetée (16) entraînée par un moteur, varie selon une fonction sinusoïdale.

5. Appareil radiologique suivant la revendication 3 ou 4, caractérisé par le fait que sur le levier de charge (10) se trouve monté un autre capteur (30), qui envoie un signal correspondant aux écarts du levier de charge (10) par rapport à la position centrale, à un autre circuit de régulation (37), qui commande le moteur (28) servant à entraîner le chariot longitudinal (26) afin de compenser l'écart.

## Claims

1. X-ray apparatus having an apparatus mounting foot (1), a tiltable section (2, 3, 26), a vehicle (3, 4) on the tiltable section (2, 3, 26) running along guide rails (8, 23) and being displaceable, for X-ray parts having weight-counterbalancing spring means, with counterbalancing forces which can be adjusted by motor each according to the position of the apparatus, characterized in that at least one slot-shaped opening (9, 27) is fitted in the vehicle (3, 4) which opening extends essentially perpendicular to the guide rails (8, 23), and in which opening the one end of a onearmed load laver (10) can be displaced, which load lever is hinged at the tiltable section (2, 3, 26), in that between its ends, the load lever (10) is connected to a junction box (13), which is disposed on a rod (12) so that it slides, so that the load lever (10) can pivot, the junction box being supported by a spring (14) on that end of the rod (12) which faces the vehicle (3, 4), the other end of the rod (12) being hinged at a nut (17) which can be adjusted by a threaded spindle (16) which can be motor-operated, the threaded spindle (16) being arranged essentially parallel to the guide rails (8, 23).

2. X-ray apparatus according to claim 1, characterized in that the weight-counterbalancing spring means for a sighting mechanism vehicle (4) is arranged in the inside of a guide tower (3).

3. X-ray apparatus according to claim 1, characterized in that the weight-counterbalancing spring means is arranged in a longitudinal vehicle (26), which can be transported by motor, within a tilting table (2).

4. X-ray apparatus according to one of claims 1 to 3, characterized in that a motor (19) is fitted on to the threaded spindle (16) which motor is connected to a control circuit (31 to 36) an in that an angle detector (21) is allocated to the apparatus mounting foot (1) and a length detector (20) is allocated to the threaded spindle (16), which are connected to the control circuit (31 to 36), so that the force which can be adjusted by the motor-driven threaded spindle (16) and which is to be counterbalanced, has a sinusoidal angle function.

5. X-ray apparatus according to claim 3 or 4, characterized in that an additional detector (30) is fitted on to the load lever (10), which detector supplies a signal, corresponding to the deviations of the load lever (10) from the middle position, to an additional control circuit (37) which controls the motor (28) for the longitudinal vehicle (26) in such a way that the deviation is counterbalanced.

FIG 1

FIG 3

FIG 2